# EUROPEAN PATENT APPLICATION

(11) **EP 4 000 518 A1**
(43) Date of publication of application: **25.05.2022**
(21) Application number: 20209651.7
(22) Date of filing: 24.11.2020
(51) Int. Cl.: A61B 5/15, A61B 5/151

(54) **TRIGGER MECHANISM FOR A SAMPLE COLLECTION DEVICE AND SAMPLE COLLECTION DEVICE INCLUDING THE SAME**

(71) Applicant: Loop Medical SA, 1095 Lutry (CH)
(72) Inventor: QUEVAL, Arthur, 1095 Lutry (CH)
(74) Representative: DTS Patent- und Rechtsanwälte Schnekenbühl und Partner mbB

(57) **Abstract**

The present invention relates to a trigger mechanism for a sample collection device (100), the trigger mechanism comprising a trigger element (400) and a preloaded plug with a blade (600), whereas the trigger element (400) is relatively movable to the plug, the trigger mechanism being configured to release the preloaded plug with the blade (600) for perforating a user's skin for blood collection, the trigger element (400) further being arranged to be activated and moved relatively to the plug by an activation force created by a skin part being vacuum-sucked into the sample collection device (100) and pressing directly or indirectly against the trigger element (400).

## Description

The present invention concerns a trigger mechanism for a sample collection device for collecting a sample of a fluid of a user, e.g. blood, in particular capillary blood. Furthermore, the present invention relates to a sample collection device including the trigger mechanism. Still further, the present invention relates to a trigger element for the trigger mechanism.

Venipuncture is a blood collection method where the vein is punctured by a hollow needle, and where blood is collected into a tube. This method allows collection of large and high-quality blood samples into tubes. Several tubes can be filled during one blood sampling. Furthermore, these tubes are compatible with highly automated blood analyzers, which can analyze thousands of samples per day. These high throughputs capabilities answer the growing need to fast and clinical grade diagnostics at the lowest cost.

However, this method requires a healthcare professional (e.g. a nurse) with a specific qualification further than a dedicated infrastructure. Moreover, risks are associated with puncturing the vein: if the vein is fragile or if the gesture is not performed properly, it can result in a hematoma. There is also a risk of needle-stick injury, which may expose the healthcare professionals to blood-borne diseases.

On the other hand, the finger prick method consists in the incision of the skin at the fingertip using a lancet. A drop or a few drops of capillary blood can be collected into capillary tubes or into dedicated analytical devices (e.g. microfluidic devices, lab-on-chip, paper-based diagnostic tools, ...). While this technique does not require highly trained professional and can be performed by the patient himself, it is very difficult to collect blood above 100 µl and to perform many analyses per sample.

Moreover, the blood collected into glass capillaries or through other devices, cannot be analyzed by automated analyzers, used by central laboratories, which require to have a minimum dead volume of blood of 100 µl to 200 µl contained into a single tube.

In some instances, more blood, up to 0.5 ml, can be collected with the finger prick method. However, this requires to press and squeeze the finger in order to collect more blood. Squeezing too hard may result in hemolysis (damage of the red blood cells) and dilution of the blood sample by the interstitial fluid, contained in spaces between the tissue cells. For these reasons, and to keep a good blood quality, the use of finger prick is generally limited to the collection of small volumes of blood.

An improved system allowing simplifying collection of a fluid of a user (e.g., blood) while keeping a high-quality standard for its analysis is known from WO2019220340, filed in the name of the same applicant. Said known extraction and collection system includes a first suction pack and a second suction pack, the first suction pack being arranged to be received by the second suction pack. Accordingly, a first chamber is defined between the first suction pack and the second suction pack, said chamber being placed under vacuum (e.g., in a manufacturing assembly line or in a healthcare facility). The second suction pack comprises a button, formed on one of its outer surfaces, and a piercing protrusion which is located below the button such that, once the user activates the button, the piercing protrusion pierces a membrane (e.g., an Aluminum membrane) provided in the first suction pack, thereby transferring the vacuum from the first vacuum chamber to a second chamber (i.e., a collection chamber).

Once the vacuum is released in the second chamber, the skin of the user is deformed and sucked inside the device. Then, a trigger mechanism releases a plug (e.g., preloaded by a spring), and cuts the skin for fluid (e.g., blood) collection.

The trigger force that necessary for activating the device is determined by different parameters such as elastic force of the plug (e.g., spring force), torque applied on the plug, vacuum level, skin elasticity, and coefficient of friction between the trigger element and the plug. Further, also the elastic modulus of the material of the components of the trigger mechanism, e.g. the material of the trigger element, influence the trigger force.

Though being independent from one another, the aforesaid parameters are however strictly linked to structural features of the device, which drastically limits the degree of freedom in adjusting the trigger force.

It may be appreciated that, whether the trigger force is too low, the sample collection device may be unwantedly activated before the skin is sufficiently sucked-in and deformed, this resulting in an insufficient skin cut (or even no skin cut at all), or during transportation, thereby rendering the device unsuitable for use.

On the other hand, whether the trigger force is too high, the achieved skin deformation may not be sufficient for activating the device.

In this regard, one possible solution could be increasing vacuum level to maximize skin deformation. Nonetheless, this would increase the risk of skin bruising. Hence, a similar solution is considered not to be acceptable.

It is an object of the present invention to provide a trigger mechanism for a sample collection device allowing achieving an appropriate modulation of the trigger force for activating the sample collection device, thereby allowing painlessly making a proper cut for blood collection.

This object is achieved by the trigger mechanism of claim 1. Advantageous embodiments of the present invention regarding the trigger mechanism are described in dependent claims 2 to 18 and also below.

According to the present invention, a trigger mechanism for a sample collection device includes:
- a trigger element, and
- a preloaded plug with a blade,
wherein the trigger element is relatively movable to the plug;
wherein the trigger mechanism is configured to release the preloaded plug with the blade for perforating a user's skin for blood collection;
further wherein the trigger element is arranged to be activated and moved relatively to the plug by an activation force created by a skin part being vacuum-sucked into the sample collection device and pressing directly or indirectly against the trigger element.

The invention is based on the basic idea that for a sample collection device, especially a blood collection device, has a triggering mechanism, which is able to unlock the blade for cutting through the skin of a patient under controlled and pre-defined conditions. In particular, it can be avoided that the trigger force is too low and thus there is a prevention of activation of the cutting mechanism and unloading of the blade carrier. Thus, the activation force is pre-defined to a level, which is corresponding to a sufficient in-sucking and thus sufficient deformation of the skin being sucked in into the sample collection device, such that a proper cut for blood collection can be made. The mechanism can generally also be used for sample collection of other fluids, but is designed for painless or low-level pain blood collection of capillary blood. In particular, the plug can be preloaded by means of a spring or the like. Further, the plug can rotate after activation such that the blade is doing its cut through the skin due to the rotational movement of its carrier, i.e. the plug.

Preferably, the trigger mechanism comprises at least one deformable and/or partially elastic element, which is configured and arranged to create at least partially a counterforce against the activation force.

The deformable and/or partially elastic element be a beam.

The beam may have a connecting end, which is connected to the main body of the trigger element, and a free end, the free end being smaller in diameter than the connecting end.

The blade has the function of a knife or a cutting element. It can be a knife or knife-like element.

Advantageously, the deformable and/or partially elastic element may have a cross section which is set to be minimum in the proximity of the end portion of the deformable and/or partially elastic element, and gradually increases towards the first end.

Accordingly, stability of the trigger element during use can be improved. Moreover, breaking of the beam upon exerting a pushing force can be largely prevented.

The trigger element has a main body, and the beam may be formed integrally on the main body of the trigger element.

This allows for easier manufacturing of the trigger mechanism.

The trigger element may be arranged eccentrically and/or being asymmetrically formed.

The trigger element may include a slot, formed between the main body of the trigger element and the beam.

Advantageously, the slot may have a first end and a second end, and a hook-shaped positioning portion may be formed in the proximity of the second end of the slot.

This aspects allow facilitating positioning of the trigger element. Moreover, stability during use can be further improved.

The trigger mechanism may include a pin, which is formed at an end portion of the deformable and/or elastic element.

The pin may be configured to be inserted into a counterstructure for positioning and/or force control purpose.

The counterstructure may be a groove, especially groove in a cap.

The trigger mechanism may be provided with a cap.

The cap may be arranged between trigger element and plug.

A spring may be arranged between cap and plug, and may be configured and arranged to pre-load the plug.

An opening may be defined between the end portion of the deformable element and the hook-shaped positioning portion.

Advantageously, the trigger element can be activated with a force between 2N and 5N.

In particular, the absolute pressure can be for example between 400 mbar and 600 mbar (adaptable range). The stroke or (elastic) deformation required to release triggering element and/or the plug/blade is reached in the embodiment described below at approx. 0.55 mm (possible range can be e.g. 0.55 mm ±10%). In other words, the deformation and/or movement of the trigger element should be approx. in the dimension (range) of approx. 0.45 mm to approx. 0.65 mm and the value of 0.55 mm was found to be very suitable. This which corresponds to a force between 2N and 5N. Test have shown that a good value is around 4N.

Accordingly, it is possible obtaining sufficient in-sucking and deformation of the skin into the sample collection device, so that a proper cut for blood collection can be made.

At the same time, undesired activation of the device before the skin is sufficiently in-sucked and deformed, or during transportation of the device, can be avoided.

The pin may be integrally formed with the deformable and/or elastic element.

The cap and the plug can be made of polypropylene and/or the trigger element can made of polycarbonate and/or high-density polyethylen (HDPE) and/or polycarbonate. The object of the present invention can further be solved by a sample collection device for extracting and collecting a sample of a fluid of a user, comprising:
- the trigger mechanism according to one or more of the above aspects;
- an outer shell;
- an inner shell having a pierceable membrane, wherein a pre-packaged vacuum is sealed in a first chamber defined between the outer shell and the inner shell, and
- a partially elastically deformable push button section, including a center portion having a sharp piercing element for piercing the pierceable membrane of the inner shell when a pushing force is applied on the push button section, for allowing releasing of the vacuum in a second chamber, defined by the inner shell,
wherein, when the vacuum is released in the second chamber and the user's skin is deformed and at least partially sucked into the sample collection device, the trigger element is configured to be activated to release the preloaded plug for perforating the user's skin for fluid (e.g., blood) collection.

Still further, the object of the present invention can be solved by a trigger element for the trigger mechanism according to one or more of the above aspects, comprising the trigger element features.

The invention will be better understood with the aid of the description of an embodiment given by way of example and illustrated by the figures, in which:
- Fig. 1: shows a cross-section view of the outer part (i.e. outer and inner shell with a piercable membrane) a sample collection device for extracting and collecting a sample of a fluid of a user, according to an embodiment of the invention (here without the trigger mechanism and the sample collection container/sample collection tube);
- Fig. 2: shows a partial cross-section view of a trigger mechanism for a sample collection device, according to an embodiment of the invention;
- Fig. 3: shows a perspective view of a cap of the trigger mechanism according to the embodiment;
- Fig. 4: shows a front view of a trigger element of the trigger mechanism according to the embodiment;
- Fig. 5: shows a perspective view of the trigger element of Fig. 4;
- Fig. 6: shows a perspective view of the detail of an elastic element of the trigger element of Fig. 4;Fig. 7a-d several views of a plug of the trigger mechanism according to the embodiment of Fig. 2;
- Fig. 8a-d: several views of a further embodiment of a plug suitable to be used for the trigger mechanism according to the embodiment of Fig, 2;
- Fig. 9a-c: further cross-sectional views of the embodiment of the plug according to Fig. 8a-d; and
- Fig. 10: a perspective view of a further embodiment of a cap.

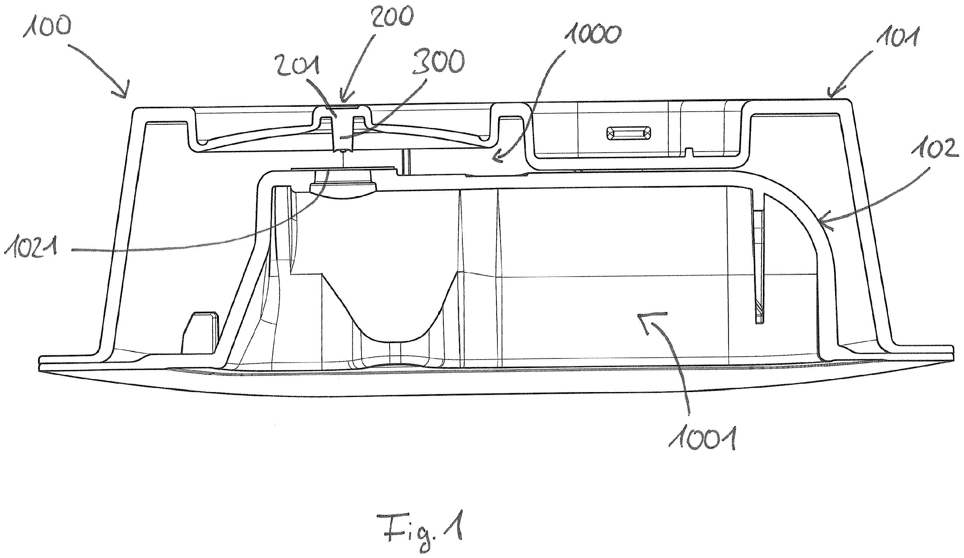

**Fig. 1** shows a cross-section view of the outer part a sample collection device 100 for extracting and collecting a sample of a fluid of a user, according to an embodiment of the invention (here without a trigger mechanism).

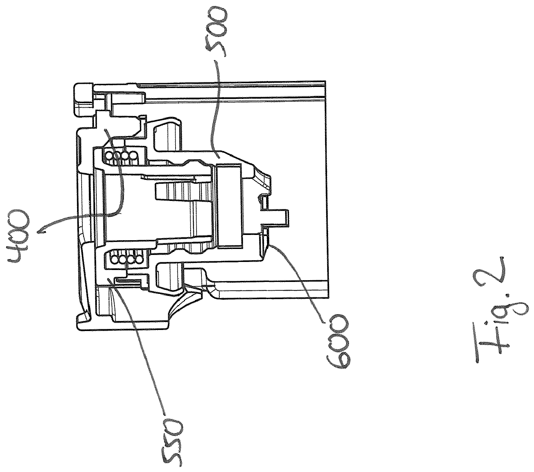

**Fig. 2** shows a trigger mechanism for the sample collection device 100, according to an embodiment of the present invention.

The trigger mechanism comprises a trigger element 400 and a preloaded plug with a blade 600. The blade 600 has the function of a knife and is intended to act as a cutting element for cutting through the skin of the patient.

The trigger element 400 is relatively movable with respect to the plug 500, and is configured to release the preloaded plug 500 with the blade 600 or knife 600 for perforating a user's skin for fluid collection (e.g., blood collection).

Specifically, the trigger element 400 is configured to be activated and moved relatively to the plug 500 by an activation force created by a skin part being vacuum-sucked into the sample collection device 100 and pressing, directly or indirectly, against the trigger element 400.

Advantageously, the trigger element 400 is configured to be activated with a force between 2N and 5N. A well-suited value would be for example 4N.

Thus, the trigger element 400 may be easily activated by the skin part that is vacuum-sucked and deformed into the sample collection device 100, and which directly or indirectly exerts a pressure against the trigger element 400.

At the same time, undesired activation of the device before the skin of the user is sufficiently in-sucked and deformed into the device 100 can be prevented.

In the illustrated embodiment, the trigger mechanism advantageously comprises at least one deformable and/or partially elastic element 402.

Specifically, the deformable and/or partially elastic element 402 is configured and arranged to create, at least partially, a counterforce against the activation force.

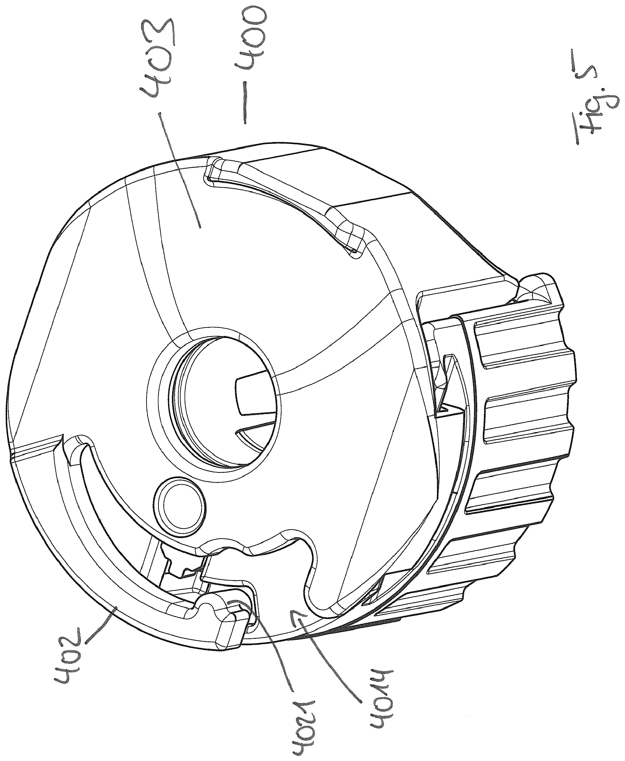

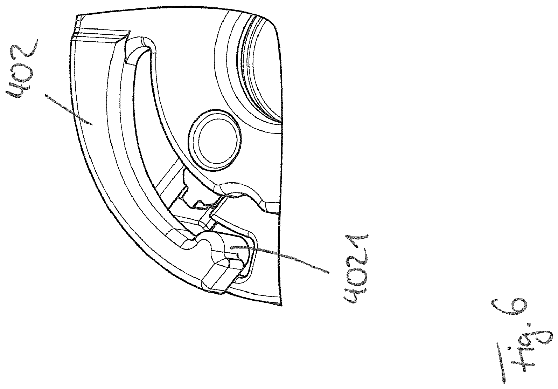

In the illustrated embodiment, the deformable and/or partially elastic element 402 is a beam 402 **(****Figs. 5-6****).**

Preferably, the beam 402 has a connecting end, which is connected to a main body 403 of the trigger element 400, and a free end, which is smaller in diameter than the connecting end. This way, the beam 402 has a connecting end, which is connected to a main body 403 of the trigger element 400, and a free end, wherein the free end has a smaller cross-section than the connecting end.

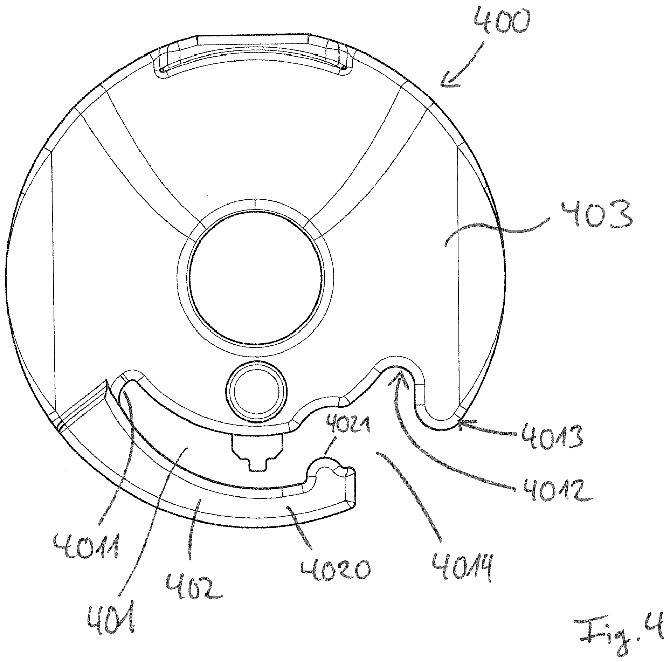

More preferably, the beam 402 has a cross section which is set to be minimum in the proximity of the end portion 4020 of the beam 402, and gradually increases towards the first end 4011 **(****Figs. 4 to 6****).**

The provision of a gradually increasing cross section of the beam 402 towards the first end 4011 allows reducing the risk of breaking of the beam 402 when a pushing force is exerted.

In the illustrated embodiment, the trigger element 400 has a main body 403, and the beam 402 is formed integrally on the main body 403.

For example, the main body 403 and the beam 402 may be integrally formed through injection molding.

The trigger element may be arranged eccentrically and/or may be asymmetrically formed.

In the illustrated embodiment, as shown in **Figs. 4 to 6****,** the trigger element 400 has a slot 401, formed between the main body 403 of the trigger element 400 and the beam 402.

Advantageously, the slot 401 has a first end 4011 and a second end 4012, and a hook-shaped positioning portion 4013 is formed in the proximity of the second end 4012 of the slot 401.

Accordingly, positioning of the trigger element 400 can be facilitated. Moreover, stability during use can be further improved.

In the illustrated embodiment, a pin 4021 is formed at an end portion 4020 of the deformable and/or elastic element 402 (cf. **Figs. 4-6**).

The pin 4021 is configured to be inserted into a counterstructure for positioning and/or force control purpose.

Preferably, the counterstructure is a groove. More preferably, the counterstructure is a groove formed in a cap 550.

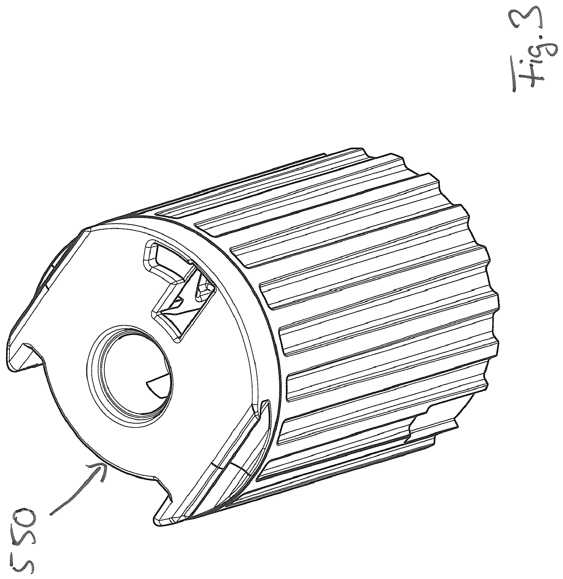

In the illustrated embodiment, the trigger mechanism 400 includes a cap 550 **(****Fig. 3****).**

The cap 550 is arranged between trigger element 400 and plug 500.

A spring may be arranged between cap 550 and plug to pre-load the plug 500.

In the illustrated embodiment, as shown in **Figs. 4-5****,** an opening 4014 is defined between the end portion 4020 of the deformable and/or partially elastic element 402 and the hook-shaped positioning portion 4013.

Advantageously, the pin 4021 may be integrally formed with the deformable and/or partially elastic element 402.

The cap 550 and the plug can be made of polypropylene.

The trigger element 400 can be made of polycarbonate. Alternatively, it can be made of HDPE and/or polycarbonate.

A part of the sample collection device 100 according to an embodiment of the invention is shown in **Fig. 1****.**

The sample collection device 100 is configured for collecting a sample of a fluid of a user, in particular a bodily fluid, e.g. blood, in particular capillary blood.

The sample collection device 100 includes the trigger mechanism described above.

Furthermore, the sample collection device 100 comprises an outer shell 101.

Still further, the sample collection device 100 comprises an inner shell 102, having a pierceable membrane 1021 (e.g., an Aluminium membrane).

A pre-packaged vacuum is sealed in a first chamber 1000, defined between the outer shell 101 and the inner shell 102.

The sample collection device 100 further comprises an elastically deformable push button section 200.

Specifically, in the illustrated embodiment, the push button section 200 includes a center portion 201 having a sharp piercing element 300 for piercing the pierceable membrane 1021 of the inner shell 102 upon applying a pushing force on the push button section 200, so as to determine releasing of the vacuum in a second chamber 1001, defined by the inner shell 102.

The release of the vacuum in the second chamber 1001 determines deformation of the user's skin, such that the skin is at least partially sucked into the sample collection device 100 and the trigger element 400 is activated to release the preloaded plug for perforating the user's skin for fluid collection (e.g., blood collection).

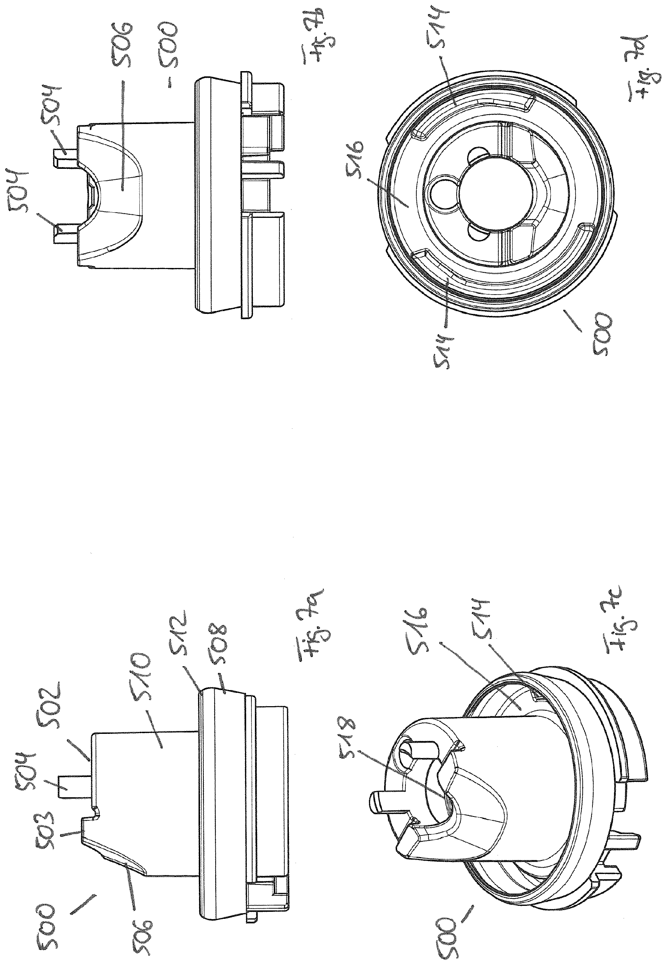

**Fig. 7a****-d** show several views of a plug 500 of the trigger mechanism according to the embodiment of **Fig. 2****.**

As can be seen Fig. 7a, which is a side view of the plug 500, the plug 500 has a mounting plateau 502 for mounting and holding the blade 600 (not shown in **Fig. 7a****-d**).

For mounting the blade 600, there are two mounting pins 504, which hold the blade 600. The pins 504 are integrally formed parts of the plug 500. It is formed by means of injection moulding.

The pins 504 are then connected and fixed to the blade for fixation of the blade to the plug 500 e.g. by means of heat-stacking and/or pressing and/or heating and/or (partially) welding or the like.

Adjacent to this and connected to the mounting plateau 502, there is chamfer section 506.

The chamfer section 506 has the following function:
During the rotation of the plug 500 with the blade 600 the chamfer is not interfering and touching the wound, which is created by the blade 600 and needed for blood collection.

Further, the plug 500 has a sealing ring 508, which is integrally formed to the other end of the plug 500 being opposite to the mounting plateau 502.

The sealing ring 508 has a collar-like form. Further, the sealing ring 508 is arranged with some distance and in parallel to the main body 510 of the plug 500. It is used to closed and seal the blood container after sample collection, by e.g. just pressing the plug into the opening of the blood sample container (not shown). The blood sample container can be e.g a cylinder-shaped tubular container (with one opening), being held by the inner shell.

The sealing is done radially with a sealing lip 512 arranged at the end of the sealing ring 508.

As can be further seen from **Fig. 7d****,** the plug 500 also comprises loading grooves 514 being formed into the connecting section 516 between the main body 510 and the sealing ring 508.

The loading grooves 514 extend in width at one end to accommodate easier with a loading tool during manufacturing.

In the middle of the main body 510 there is a septum 518 made of e.g. an elastomer (or any other suitable material). The septum can be mounted to the plug 500 by means of e.g. overmoulding or press-fit or any other suitable manufacturing method.

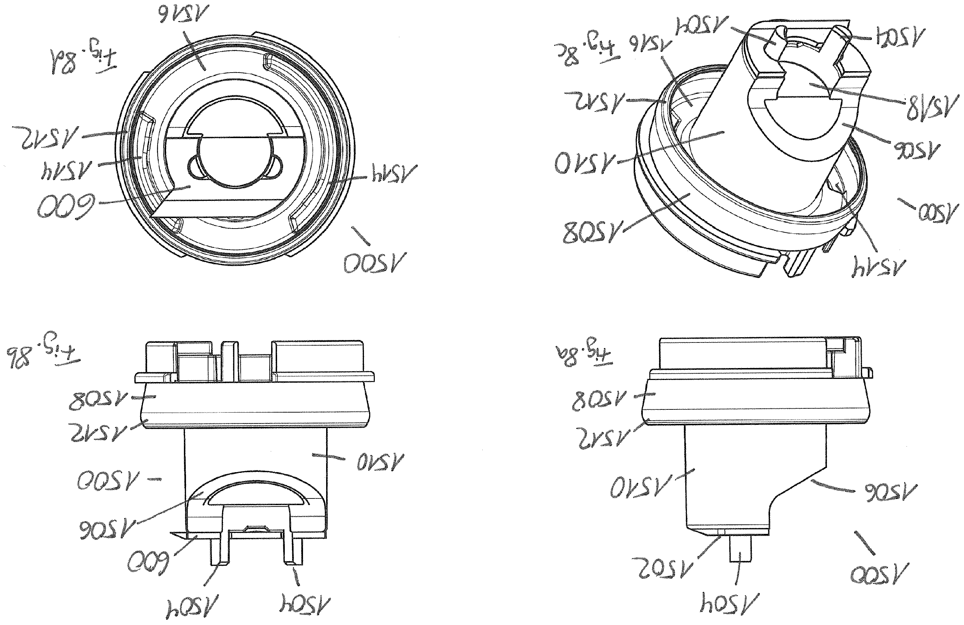

**Fig. 8a****-d** show several views of a further embodiment of a plug 1500 suitable to be used for the trigger mechanism according to the embodiment of **Fig. 2****.**

The plug 1500 has the identical and/or similar feature in terms of structure and function like the plug 500 shown in **Fig. 7a****-d.** The same elements/features are therefore denotated with the same reference number, but increased in value with thousand (value + 1000).

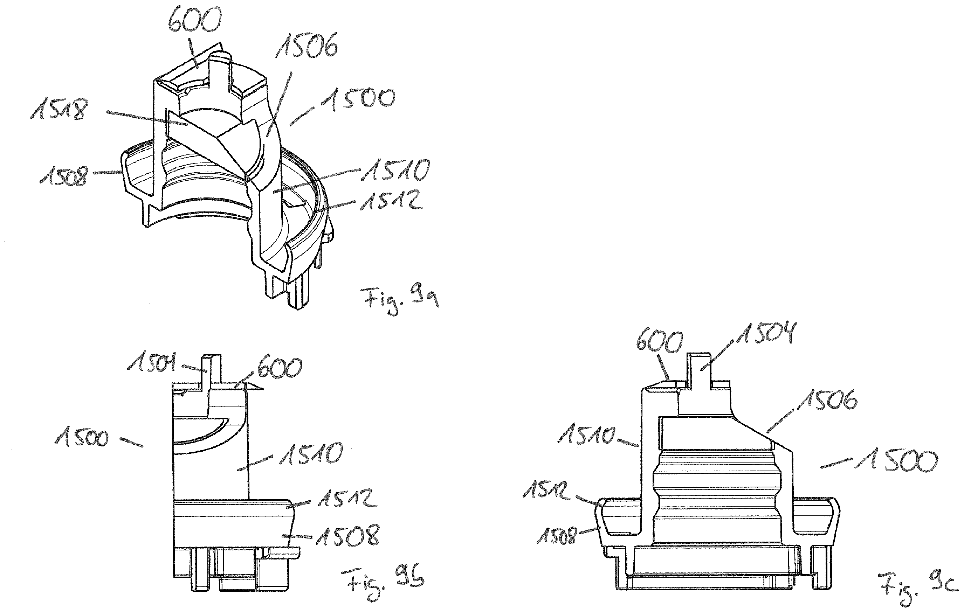

**Fig. 9a****-c** show further cross-sectional views of the embodiment of the plug according to **Fig. 8a****-d.**

### The following difference exists:

The chamfer section 1506 is increased and more material is "cut away" from the main body 1510. By this, the possible interference of the plug 1500 with the wound can be further reduced and the possible blood collection volume during the time frame of blood collection can be increased.

In the shown embodiment of **Fig. 8a****-c,** the chamfer section 1506 extends from the outer wall of the main body 1510 to almost the middle line of the main body 1510, as can be e.g. seen from **Fig. 8d****.**

The angle of the chamfer is here chosen in the range 15°-40°, here approx. 30°.

Also the chamfer section 1506 ends below the mounting plateau 1502 and thus has a chamfer-step form.

In the angle of the chamfer according to the embodiment shown in **Fig. 7a****-d** is much steeper, and chosen in the range of approx. 65°-80°, here approx. 75°.

Also, there is no step in the chamfer section 506.

The chamfer of the chamfer section 506 extends from the outside of the main body 510 to the mounting plateau 502.

There are on the mounting plateau 502 two shoulders 503.

The chamfer extends to the shoulders 503, which are arranged half the way to the inside from the outer wall toward the middle line of the main body 510.

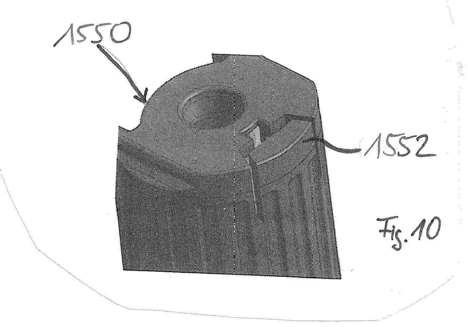

**Fig. 10** shows a perspective view of a further embodiment of a cap 1550. The cap 1550 has all the structural and functional features as the cap 550 describe above.

### The following difference exists:

The cap 1550 comprises a deformable and/or elastic element 1552, which is integrally formed to the cap 1550.

It has the function of the deformable and/or elastic element 402 as described above. The overall device is then adapted accordingly.

### Reference signs used in the figures

- 100: Sample collection device
- 101: Outer shell
- 102: Inner shell
- 200: Push button section
- 300: Piercing element
- 201: Center portion of the push button section
- 400: Trigger element
- 401: Slot of the trigger element
- 402: Deformable and/or elastic element
- 403: Main body of the trigger element
- 500: Plug
- 502: Mounting plateau
- 503: Shoulder
- 504: Pin
- 506: Chamfer Section
- 508: Sealing Ring
- 510: Main body
- 512: Sealing lip
- 514: Loading Groove
- 516: Connecting Section
- 518: Septum
- 550: Cap
- 600: Blade // Knife
- 1000: First chamber
- 1001: Second chamber
- 1021: Pierceable membrane of the inner shell
- 1500: Plug
- 1502: Mounting plateau
- 1504: Pin
- 1506: Chamfer section
- 1508: Sealing ring
- 1510: Main body
- 1512: Sealing lip
- 1514: Loading Groove
- 1516: Connecting Section
- 1518: Septum
- 1550: Cap
- 1552: Deformable and/or elastic element
- 4011: First end of the slot
- 4012: Second end of the slot
- 4013: Hook-shaped positioning portion
- 4014: Opening
- 4020: End portion of the deformable element
- 4021: Pin

## Claims

1. A trigger mechanism for a sample collection device (100), the trigger mechanism comprising a trigger element (400) and a preloaded plug (500) with a blade (600), whereas the trigger element (400) is relatively movable to the plug (500), the trigger mechanism being configured to release the preloaded plug with the blade (600) for perforating a user's skin for blood collection, the trigger element (400) further being arranged to be activated and moved relatively to the plug by an activation force created by a skin part being vacuum-sucked into the sample collection device (100) and pressing directly or indirectly against the trigger element (400).

2. The trigger mechanism according to claim 1, **characterized in that** the trigger mechanism comprises at least one deformable and/or partially elastic element (402), which is configured and arranged to create at least partially a counterforce against the activation force.

3. The trigger mechanism according to claim 2, **characterized in that** the deformable and/or partially elastic element (402) is a beam (402).

4. The trigger mechanism according to claim 3, **characterized in that** the beam (402) has a connecting end, which is connected to a main body (403) of the trigger element (400), and a free end, wherein the free end has a smaller cross-section than the connecting end.

5. The trigger mechanism according to claim 4, **characterized in that** the deformable and/or elastic element (402) has a cross section which is set to be minimum in the proximity of the end portion (4020) of the deformable and/or partially elastic element (402), and gradually increases towards the first end (4011).

6. The trigger mechanism according to claim 4 or claim 5, **characterized in that** the trigger element has a main body (403) and the beam (402) is formed integrally on the main body (403).

7. The trigger mechanism according to one of the preceding claims, **characterized in that** the trigger element (400) is arranged eccentrically and/or is asymmetrically formed.

8. The trigger mechanism according to one of the preceding claims, **characterized in that** the trigger element (400) has a slot (401) formed between the main body (403) of the trigger element (400) and the beam (402).

9. The trigger mechanism according to claim 8, **characterized in that** the slot (401) has a first end (4011) and a second end (4012), wherein a hook-shaped positioning portion (4013) is formed in the proximity of the second end (4012) of the slot (401).

10. The trigger mechanism according to one of claims 2 to 9, **characterized in that** a pin (4021) is formed at an end portion (4020) of the deformable and/or elastic element (402) and is configured to be inserted into a counterstructure for positioning and/or force control purpose.

11. The trigger mechanism according to claim 10, **characterized in that** the counterstructure is groove, especially groove in a cap (550).

12. The trigger mechanism according to one of the preceding claims, **characterized in that** the trigger mechanism has a cap (550).

13. The trigger mechanism according to claim 12, **characterized in that** the cap (500) is arranged between trigger element (400) and plug.

14. The trigger mechanism according to claim 12 or claim 13, **characterized in that** between cap (500) and plug a spring is arranged, whereas the spring is configured and arranged to pre-load the plug.

15. The trigger mechanism according to one of claims 2 to 14, **characterized in that** an opening (4014) is defined between the end portion (4020) of the deformable and/or partially elastic element (402) and the hook-shaped positioning portion (4013).

16. The trigger mechanism according to one of the preceding claims, **characterized in that** the trigger element (400) is configured to be activated with a force between 2N and 5N.

17. The trigger mechanism according to one of the preceding claims, **characterized in that** the pin (4021) is integrally formed with the deformable and/or partially elastic element (402).

18. The trigger mechanism according to one of the preceding claims, **characterized in that** the cap (500) and the plug are made of polypropylene and/or the trigger element (400) is made of HDPE and/or polycarbonate.

19. A sample collection device (100) for extracting and collecting a sample of a fluid of a user, the sample collection device (100) comprising:
- the trigger mechanism according to any one of the preceding claims;
- an outer shell (101);
- an inner shell (102) having a pierceable membrane (1021), wherein a pre-packaged vacuum is sealed in a first chamber (1000) defined between the outer shell (101) and the inner shell (102), and
- a partially elastically deformable push button section (200), including a center portion (201) having a sharp piercing element (300) for piercing the pierceable membrane (1021) of the inner shell (102) when a pushing force is applied on the push button section (200), for allowing releasing of the vacuum in a second chamber (1001), defined by the inner shell (102),
wherein, when the vacuum is released in the second chamber (1001) and the user's skin is deformed and at least partially sucked into the sample collection device (100), the trigger element (400) is configured to be activated to release the preloaded plug for perforating the user's skin for fluid collection.

20. A trigger element for a trigger mechanism according one of claims 1 to 18, comprising the trigger element features.
